(19) Europäisches Patentamt
European Patent Office
Office européen des brevets

(11) Veröffentlichungsnummer: 0 088 718
B1

# (12) EUROPÄISCHE PATENTSCHRIFT

(45) Veröffentlichungstag der Patentschrift: 18.10.89

(21) Anmeldenummer: 83710004.9

(22) Anmeldetag: 18.02.83

(51) Int. Cl.⁴: C 08 G 65/28, C 08 G 65/06, C 08 G 65/32, C 11 D 3/37, B 01 F 17/00, C 09 K 3/20

## (54) Verdickungsmittel auf der Basis Polyätherderivate.

(30) Priorität: 03.03.82 DE 3207613
26.01.83 DE 3302465

(43) Veröffentlichungstag der Anmeldung:
14.09.83 Patentblatt 83/37

(45) Bekanntmachung des Hinweises auf die Patenterteilung:
18.10.89 Patentblatt 89/42

(84) Benannte Vertragsstaaten:
AT BE CH FR GB IT LI NL SE

(56) Entgegenhaltungen:
EP-A- 0 018 482
EP-A- 0 024 472
US-A- 4 288 639
US-A- 4 312 775

**Die Akte enthält technische Angaben, die nach dem Eingang der Anmeldung eingereicht wurden und die nicht in dieser Patentschrift enthalten sind.**

(73) Patentinhaber: Akzo Patente GmbH,
Postfach 10 01 49 Kasinostrasse 19-23,
D-5600 Wuppertal-1 (DE)

(72) Erfinder: Seibert, Karl, Dipl.-Ing., Am Hinzenbusch 62,
D-5160 Düren-Niederau (DE)
Erfinder: Neumann, Klaus, Appolinarisstrasse 71,
D-5166 Kreuzau-Obermaubach (DE)
Erfinder: Spiess, Ernst, Dipl.-Ing., Schulstrasse 20,
D-5042 Erftstadt-Gymnich (DE)
Erfinder: Brull, Hendrik G., Dr. Dipl.-Chem., Irenelaan 41,
NL-6713 Ede/Niederlande (NL)

(74) Vertreter: Schalkwijk, Pieter Cornelis, Akzo Patents Department P.O. Box 314, NL-6800 AH Arnhem (NL)

EP 0 088 718 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft Verdickungsmittel auf der Basis Polyätherderivate. Verdickungsmittel werden für die Verdickung von natürlichen und synthetischen Ölen, wäßrigen Elektrolytlösungen und wäßrigen Lösungen organischer Substanzen eingesetzt. Dadurch soll beispielsweise die Handhabbarkeit von Flüssigkeiten, die beispielsweise als Haushaltschemikalien eingesetzt werden, verbessert werden. Auch bei Elektrolytlösungen, die in Batterien benötigt werden ist es üblich, Verdickungsmittel zu verwenden. Bisher hat man als Verdickungsmittel Wachse, Carboxyalkylcellulosen, Stärke, Xanthan-Gum oder hochmolekulare Ester eingesetzt.

Bei kosmetischen tensidhaltigen Zubereitungen zur Stabilisierung disperser Systeme und zur besseren Handhabung bei der Anwendung ist es üblich, Verdickungsmittel einzusetzen. Bei Äthersulfaten ist es allgemein üblich, die Zubereitungen mit anorganischen Elektrolyten, vorzugsweise Kochsalz, zu verdicken. Bei anderen Tensiden werden beispielsweise Distearylpolyglykolester, Cellulosederivate, natürliche und synthetische lösliche Polymere eingesetzt. Die so erhaltenen Zubereitungen zeigen ein im wesentlichen strukturviskoses und/oder thixotropes Verhalten.

Nach DIN 53900 versteht man unter Tensiden chemische Verbindungen, die, in einer Flüssigkeit gelöst oder dispergiert, an einer Grenzfläche bevorzugt adsorbiert werden; dadurch wird eine Anzahl von praktisch bedeutungsvollen physikalisch-chemischen oder chemischen Eigenschaften hervorgerufen. Synonym zu Tensid wird auch von einer grenzflächenaktiven Verbindung gesprochen. Tensidhaltige Zubereitungen sind Gemische, die Tenside als wesentliche Bestandteile enthalten.

Aus US-A-4 288 639 sind Polyäther bekannt, welche durch Reaktion von Äthylenoxid mit einem monohydrischen Alkohol mit 12 bis 18 Kohlenstoffatomen und durch anschließende Reaktion von diesem Homopolymerisat mit einem α-Olefinoxid mit einer Kettenlänge von 12 bis 18 Kohlenstoffatomen hergestellt werden. In den Beispielen werden Polymerisate mit Molekulargewichten von 8717 bis 13095, welche sowohl Äthylenoxid-Reste als auch Propylenoxid-Reste enthalten, erläutert. Die EP-A-24 472 beschreibt zur Erniedrigung der Grenzflächenspannung öliger Phasen gegen Wasser geeignete Polyäther. Diese Polyäther sind praktisch nicht mehr wasserlöslich.

Aufgabe der vorliegenden Erfindung war es ein Verdickungsmittel zu finden, welches im Viskositätsverhalten sich wie eine Newtonsche Flüssigkeit verhält und außerdem unter den üblichen Verwendungsbedingungen hydrolysestabil ist. Gelöst wird diese Aufgabe durch einen Polyäther der allgemeinen Formel

$$R_1O(CH_2CH_2O)_n(CH_2\underset{\displaystyle R_2}{\underset{|}{C}}HO)_pH$$

wobei

$R_1$ ein substituierter oder unsubstituierter aliphatischer Kohlenwasserstoffrest mit 10–22 C-Atomen oder ein Alkylphenylrest mit einer Alkylkette von 7–15 Kohlenstoffatomen,

$R_2$ ein Alkylrest mit 8–30 Kohlenstoffatomen,

n 40–80 und

p 1–3 ist.

Vorzugsweise ist die Äthergruppe ($R_1$) ein Talgrest. Vorzugsweise ist $R_2$ ein Alkylrest mit 10–18 Kohlenstoffatomen.

In der allgemeinen Formel der erfindungsgemäßen Polyäther hat p bevorzugt den Wert 1.

Die erfindungsgemäßen Polyäther haben bevorzugt ein mittleres Molekulargewicht im Bereich von 2150 bis 3400.

Die erfindungsgemäßen Polyäther lassen sich herstellen durch stufenweise Anlagerung eines langkettigen 1,2 Epoxides mit einer Kettenlänge von 10–32 C-Atomen bei Temperaturen von 100 bis 200 °C an Polyäthylenglykolmonoäther, deren Äthergruppe ein substituierter oder unsubstituierter aliphatischer Kohlenwasserstoffrest mit 10–22 C-Atomen oder ein Alkylphenylrest mit einer Alkylkette von 7–15 Kohlenstoffatomen ist, unter Verwendung von 0,1 bis 1 Gew.-% Alkalihydroxid, bezogen auf die Gesamtmenge der Ausgangsstoffe. Die Herstellung erfolgt analog den bekannten Umsetzungen von Alkoholen mit 1,2 Epoxiden, wie sie in Houben-Weyl, Methoden der organischen Chemie (1965) Band 14,2; Seiten 436–450 beschrieben sind.

Das erfindungsgemäße Verdickungsmittel wird für wasserfreie flüssige Systeme, wie beispielsweise Polyalkylenglykole, spezielle Fettsäureester wie zum Beispiel Trimethylolpropanoleat oder Diisodecyladipat, organische Salze wie beispielsweise Diäthanolaminsalze, oder pflanzliche Öle wie zum Beispiel Sojaöl oder Rizinusöl, für tensidfreie wäßrige Lösungen wie zum Beispiel Säurelösungen, Elektrolytlösungen oder Alkohollösungen, oder für tensidhaltige Zubereitungen verwendet, wobei der Gehalt an Polyätherderivat in der tensidhaltigen Zubereitung 0,5 bis 10 Gew.-%, bezogen auf das Gewicht der Zubereitung beträgt. Die tensidhaltige Zubereitung kann als Haushaltreiniger, kosmetische Emulsion oder kosmetisches Gel, Frisierpräparat, Schaumbad oder dergleichen eingesetzt werden. Tensidhaltige Zubereitungen mit dem erfindungsgemäßen Polyätherderivat können auch in Hydraulikflüssigkeiten, in Bearbeitungsflüssigkeiten zur spanlosen und spanabhebenden Bearbeitung von Werkstoffen und ähnlichem eingesetzt werden.

Die Konzentration von Tensiden in das erfindungsgemäße Polyätherderivat enthaltenden tensidhaltigen Zubereitungen beträgt vorzugsweise 1 bis 40 Gew.-%. Dabei wirken die Tenside zum Teil auch in der Weise, daß das Tensid das Verdickungsmittel solubilisiert. Das gilt insbesondere bei solchen tensidhaltigen Zubereitungen, die als Hydraulikflüssigkeiten oder als Bearbeitungsflüssigkeiten zur spanlosen und spanabhebenden Bearbeitung von Werkstoffen eingesetzt werden.

Außer den bereits erwähnten Anwendungsgebieten werden tensidhaltige Zubereitungen mit Verdickungsmitteln in der Nahrungsmittelindustrie, vorzugsweise zum Verzögern der Zuckerkristallisation oder um Geschmacksstoffe zu fixieren, verwendet. Andere Anwendungsgebiete liegen in der Papierindustrie und in der Textilindustrie.

Tensidfreie und tensidhaltige Haushaltschemikalien können zur Verbesserung der Handhabung mit den erfindungsgemäßen Verdickungsmitteln zu höheren Viskositäten verdickt werden. Beispielsweise sind diese Säuren zur Reinigung im Sanitärbereich, Alkohollösungen als Reinigungs- und Pflegemittel und Abbeizmittel für Anstriche. Selbstverständlich sind die erfindungsgemäßen Verdickungsmittel auch bei tensidfreien Zubereitungen in der Nahrungsmittelindustrie einsetzbar, wie auch bei Elektrolytlösungen für Batterien oder pflanzlichen Ölen, wie Rizinusöl oder Sojaöl.

Die nachfolgenden Beispiele dienen der Erläuterung der Erfindung.

Beispiel 1

Herstellung der erfindungsgemäßen Verdickungsmittel.

Mit einem Dreihalskolben mit Rührer und absteigendem Kühler werden 0,2 Mol eines Fettalkoholäthoxylats der allgemeinen Formel $R_1O(CH_2-CH_2O)_nH$ in der Schmelze bei 80 °C mit 0,5% 90%igem Kaliumhydroxid-Pulver (bezogen auf die Gesamtmenge) versetzt. Es wird während 0,5 h unter Durchleiten von Stickstoff bei 80 °C gehalten. Danach wird unter Wasserstrahlvakuum von 12 bis 14 Torr (16–19 mbar) auf 140 °C erhitzt und während 0,5 h im Vakuum gehalten. Nach Entspannen mit Stickstoff wird während einer Stunde 0,2 Mol Epoxyalkan zugetropft. Nach einer Nachreaktionszeit von 2 Stunden ist die Epoxidreaktion beendet. Das Endprodukt wird nach Abkühlen auf 80 °C mit Eisessig auf $P_H$ 6,5 bis 7 eingestellt. Die nach diesem Verfahren hergestellten Produkte sind in der nachfolgenden Tabelle mit ihren Eigenschaften zusammengestellt.

Tabelle 1

| Produkt | $R_1$ | n | $R_2$ | mittleres Molekular-gewicht | Fließbereich | Aussehen |
|---|---|---|---|---|---|---|
| A | Oleyl | 40 | Decyl | 2450 | 44–45 °C | gelbl. Wachs |
| B | Nonylphenol | 50 | Decyl | 2150 | 43–45 °C | bräunl. Wachs |
| C | Talgrest | 60 | Dodecyl | 2800 | 48 °C | helles Wachs |
| D | Talgrest | 80 | Decyl | 3400 | 49–51 °C | gelbl. Wachs |

Talgrest = Gemisch gesättigter Kohlenwasserstoffreste mit 16 bis 18 Kohlenstoffatomen.

Beispiel 2

Von den im Beispiel 1 hergestellten Verdickungsmitteln werden tensidhaltige Zubereitungen mit verschiedenen Tensiden, deren chemische Zusammensetzung sich aus der Zusammenstellung in Tabelle 3 ergibt, hergestellt und deren Viskosität bei 20 °C mit einem Couette-Rotations-Viskosimeter Haake CV 100 bestimmt.

Zum Vergleich wurden als bekannte Verdickungsmittel Natriumchlorid und Polyäthylenglykol-6000-distearat herangezogen – Beispiele 2j, k, s und t. Das Beispiel 2r und t zeigt den Unterschied des erfindungsgemäßen Verdickungsmittels zum bekannten Polyäthylenglykol-6000-distearat hinsichtlich der Lagerfähigkeit. Bei beiden Produkten wurden vergleichbare tensidhaltige Zubereitungen nach 25 Tagen Lagerung bei 50 °C erneut gemessen. Beim erfindungsgemäßen Produkt blieb die Viskosität praktisch konstant, während sie beim Vergleichsprodukt auf etwa 1/20 der ursprünglichen Viskosität zurückging.

Die Ergebnisse der Viskositätsmessungen sind für die Beispiele 2a bis t in der Tabelle 2 zusammengestellt, aus der auch die Zusammensetzung der jeweiligen tensidhaltigen Zubereitung hervorgeht.

Fig. 1 zeigt die Abhängigkeit der Viskosität von der Schergeschwindigkeit für die Beispiele 2k ≙ Kurve A und 21 ≙ Kurve B. Die Kurven zeigen, daß bei Verwendung der erfindungsgemäßen Verdickungsmittel bei tensidhaltigen Zubereitungen das rheologische Verhalten newtonsch ist, während bei Verwendung von Kochsalz entsprechende tensidhaltige Zubereitungen ein strukturviskoses Verhalten zeigen. Da Verdickungsmittel dazu dienen, tensidhaltige Zubereitungen mit höherer Viskosität zu erhalten, ist es natürlich nicht erwünscht, daß die Viskosität bei höheren Schergeschwindigkeiten abnimmt.

Tabelle 2

| Beispiel | Tensid (vgl. Tab.3) | Konz. Tensid % | Verdickungs-mittel | Verh. Verd.:Ten. | Viskosit. (20 °C) mPa s | bei einer Schergeschw. s−1 | rheolog. Verhalten |
|---|---|---|---|---|---|---|---|
| a | R | 7 | C | 1:1,4 | 1300 | 50 | newtonsch |
| b | R | 21 | C | 1:4,2 | 6500 | 20 | newtonsch |
| c | R | 14 | C | 1:3,5 | 620 | 150 | newtonsch |
| d | R | 14 | C | 1:1,75 | 44000 | 3 | newtonsch |

Tabelle 2 (Fortsetzung)

| Beispiel | Tensid (vgl. Tab.3) | Konz. Tensid % | Verdickungsmittel | Verh. Verd.:Ten. | Viskosit. (20 °C) mPa s | bei einer Schergeschw. s−1 | rheolog. Verhalten |
|---|---|---|---|---|---|---|---|
| e | R | 2 | C | 2,5:1 | 30 | 300 | newtonsch |
| f | R | 14 | D | 1:3,5 | 450 | 150 | newtonsch |
| g | S | 18 | B | 1:2,6 | 160 | 90 | newtonsch |
| h | T | 15 | A | 1:2,5 | 990 | 90 | newtonsch |
| i | R | 14 | – | – | 3 | 300 | newtonsch |
| j | R | 14 | NaCl | 1:3,5 | 9 | 300 | newtonsch |
| k | U | 14 | NaCl | 1:4,7 | 2000 | 30 | strukturv. |
| l | U | 14 | C | 1:2,8 | 3500 | 30 | newtonsch |
| m | V | 14 | C | 1:2,8 | 700 | 150 | newtonsch |
| n | W | 14 | C | 1:2,8 | 3500 | 30 | newtonsch |
| o | X | 14 | C | 1:2,8 | 250 | 150 | newtonsch |
| p | Y | 14 | C | 1:2,8 | 250 | 150 | newtonsch |
| q | R | 14 | C | 1:2,3 | 14400 | 12 | newtonsch |
| r | R | 14 | C | 1:2,3 | 14300 | 12 | newtonsch* |
| s | R | 14 | Z | 1:2,3 | 3290 | 60 | newtonsch |
| t | R | 14 | Z | 1:2,3 | 170 | 60 | newtonsch* |

*nach 25 Tagen Lagerung bei 50 °C

Tabelle 3

| Tensid | Typ | |
|---|---|---|
| R | anionisch | Alpha-Olefinsulfonat (Natriumsalz) auf Basis eines Alpha-Olefins $C_{14}$-$C_{16}$ |
| S | anionisch | Sulfobernsteinsäuremonoester (Dinatriumsalz) auf Basis eines äthoxylierten Fettsäurealkylols |
| T | nichtionogen/ kationisch | Dimethylkokosaminoxid |
| U | anionisch | Alkyläthersulfat (Natriumsalz) auf Basis eines äthoxylierten Fettalkohols $C_{12}$-$C_{15}$ mit 2,5 Mol Äthylenoxid |
| V | anionisch | Alkylsulfat (Triethanolaminsalz) auf Basis eines Fettalkohols $C_{12}$-$C_{14}$ |
| W | amphoter Betain-Typ | Fettsäureamidopropyl-dimethylamino-essigsäure |
| X | nichtionogen | Sorbitanmonolaurat, äthoxyliert mit ca. 20 Mol EO |
| Y | kationisch | Alkyldimethylbenzylammoniumchlorid auf Basis $C_{12}$-$C_{14}$ |

Verdickungsmittel

| | |
|---|---|
| Z | Polyäthylenglykol-6000-distearat |

Beispiel 3

Zu wäsrigen Lösungen von Salzsäure, Schwefelsäure, ortho-Phosphorsäure, Zitronensäure, Kochsalz, Calciumchlorid, Harnstoff, Äthylenglykol, Glyzerin und D(-)Sorbit, die auf 75 °C aufgeheizt wurden, wurde das in Beispiel 1 (Tabelle 1) als Produkt C *bezeichnete* Verdickungsmittel, das bei 75 °C vorher aufgeschmolzen war, unter Rühren zugegeben.

Nach Abkühlen auf Raumtemperatur sind die Lösungen wasserklar, stabil und viskos.

Die viskosen wäßrigen Lösungen von Äthanol und Isopropanol wurden wie folgt hergestellt:

Geschmolzenes in Beispiel 1 (Tabelle 1) als Produkt C bezeichnetes Verdickungsmittel von 75 °C wurde unter Rühren zu Wasser von 75 °C gegeben. Nach Abkühlen bis auf ca. 30 °C wurde Äthanol bzw. Isopropanol unter Rühren zugegeben. Nach Abkühlen bis auf Raumtemperatur sind die Lösungen wasserklar, stabil und viskos.

Die Viskosität der wäßrigen, viskosen Systeme wurde bei 25 °C mit einem KPG-Ubbelohde-Viskosimeter mit hängendem Kugelniveau. DIN 51562, gemessen. Die kinematische Viskosität wird ausgedrückt in cST.

Die Zusammensetzungen der Gemische und die Ergebnisse der Viskositätsmessungen sind in der Tabelle 4 zusammengestellt.

Tabelle 4
Verdickung von wässerigen Lösungen von Elektrolyten und Alkoholen mit Verdickungsmittel C (gemäß Tabelle 1)

| Beispiele | | zu verdickende Lösung | Konz. der Lösung % | Konzentration Verdickungsm. % | Viskosität cSt, 25 °C (1cSt ≙ $10^{-2}cm^2/s$) |
|---|---|---|---|---|---|
| | | Wasser | – | 10 | 250 |
| I | | Salzsäure | 10 | – | 1,02 |
| I | a) | Salzsäure | 10 | 10 | 215 |
| II | | Schwefelsäure | 10 | – | 1,08 |
| II | a) | schwefelsäure | 10 | 10 | 823 |
| III | | ortho-Phosphorsäure | 10 | – | 1,17 |
| III | a) | ortho-Phosphorsäure | 10 | 10 | 810 |
| IV | | Zitronensäure | 9 | – | 1,08 |
| IV | a) | Zitronensäure | 9 | 9 | 90 |
| IV | | | | | |
| IV | b) | Zitronensäure | 18 | – | 1,31 |
| IV | c) | Zitronensäure | 18 | 9 | 115 |
| IV | d) | Zitronensäure | 27 | – | 1,63 |
| IV | e) | Zitronensäure | 27 | 9 | 170 |
| V | | Natriumchlorid | 10 | – | 1,01 |
| V | a) | Natriumchlorid | 10 | 10 | 425 |
| VI | | Kalziumchlorid | 10 | – | 1,11 |
| VI | a) | Kalziumchlorid | 10 | 10 | 400 |
| VII | | Harnstoff | 10 | – | 0,97 |
| VII | a) | Harnstoff | 10 | 10 | 823 |
| VIII | | Äthanol | 9 | – | 1,27 |
| VIII | a) | Äthanol | 9 | 9 | 72 |
| VIII | b) | Äthanol | 18 | – | 1,72 |
| VIII | c) | Äthanol | 18 | 9 | 50 |
| IX | | Propanol-2 | 9 | – | 1,38 |
| IX | a) | Propanol-2 | 9 | 9 | 108 |
| IX | b) | Propanol-2 | 18 | – | 2,01 |
| IX | c) | Propanol-2 | 18 | 9 | 51 |
| X | | Äthylenglykol | 9 | – | 1,11 |
| X | a) | Äthylenglykol | 9 | 9 | 66 |
| X | b) | Äthylenglykol | 18 | – | 1,34 |
| X | c) | Äthylenglykol | 18 | 9 | 72 |
| XI | | Glyzerin | 9 | – | 1,12 |
| XI | a) | Glyzerin | 9 | 9 | 57 |
| XI | b) | Glyzerin | 18 | – | 1,45 |
| XI | c) | Glyzerin | 18 | 9 | 75 |
| XII | | D(-)Sorbit | 9 | – | 1,14 |
| XII | a) | D(-)Sorbit | 9 | 9 | 81 |
| XII | b) | D(-)Sorbit | 18 | – | 1,48 |
| XII | c) | D(-)Sorbit | 18 | 9 | 134 |

Beispiel 4

Die in Tabelle 5 mit W1–W8 bezeichneten Flüssigkeiten wurden mit dem in Beispiel 1 (Tabelle 1) als C bezeichneten Verdickungsmittel verdickt, indem zwischen 70 und 90 °C das Verdickungsmittel unter Rühren fein dispergiert wurde. Nach Abkühlen bis auf Raumtemperatur waren zwar trübe aber stabile und viskose Dispersionen entstanden. Die Viskosität dieser nicht-wäßrigen stabilen und viskosen Dispersionen wurden ebenso wie die entsprechenden Flüssigkeiten ohne den Zusatz des Verdickungsmittels bei 25 °C mit einem Brookfield synchro-lectricviscometer, Model LVT, gemessen.

Die dynamische Viskosität wird ausgedrückt in cP. Die Ergebnisse sind ebenfalls in Tabelle 5 zusammengestellt.

Tabelle 5

| Versuch | | Flüssigkeiten | Konz. Verdickungsmittel (%) | Viskosität (Brookfield) cP. 25 °C (1cP ≙ $10^{-3}$ Pas) | UpM | Spindel |
|---|---|---|---|---|---|---|
| I | W1 | Polyäthylenglykol | – | 95 | 12 | 4 |
| Ia | | 400 | 5 | 10 000 | 12 | 4 |
| II | W2 | Polyäthylenglykol | – | 100 | 12 | 4 |
| IIa | | 400-Monoester einer isomeren Ölsäure | 5 | 3 500 | 12 | 4 |
| III | W3 | Diäthanolaminsalz einer | – | 1 040 | 12 | 4 |
| IIIa | | isomeren Ölsäure | 5 | 20 000 | 12 | 4 |
| IV | W4 | Trimethylolpropan- | – | 190 | 12 | 4 |
| IVa | | oleat | 5 | 12 500 | 12 | 4 |
| V | W5 | Trimethylolpropanester | – | 30 | 12 | 3 |
| Va | | mit $C_8$-$C_{10}$-Fettsäuren | 5 | 1 900 | 12 | 3 |
| VI | W6 | Diisodecyladipat | – | 16 | 12 | 3 |
| VIa | | | 5 | 1 700 | 12 | 3 |
| VII | W7 | Sojaöl Typ SEH | – | 50 | 12 | 4 |
| VIIa | | | 5 | 1 500 | 12 | 4 |
| VIII | W8 | Rizinusöl | – | 625 | 12 | 4 |
| VIIIa | | DIN 55939 Marke DRO | 5 | 18 500 | 12 | 4 |

**Patentansprüche**

1. Polyäther der allgemeinen Formel

$$R_1O(CH_2CH_2O)_n(CH_2\underset{\substack{|\\ R_2}}{C}HO)_pH$$

wobei

$R_1$ ein substituierter oder unsubstituierter aliphatischer Kohlenwasserstoffrest mit 10-22 C-Atomen oder ein Alkylphenylrest mit einer Alkylkette von 7–15 Kohlenstoffatomen,

$R_2$ ein Alkylrest mit 8–30 Kohlenstoffatomen,

n 40–80 und

p 1–3 ist.

2. Polyäther nach Anspruch 1, wobei $R_1$ ein Talgrest ist.

3. Polyäther nach einem der Ansprüche 1 oder 2, wobei $R_2$ ein Alkylrest mit 10–18 Kohlenstoffatomen ist.

4. Polyäther nach einem der vorangegangenen Ansprüche, wobei p den Wert 1 hat.

5. Polyäther nach Anspruch 4, mit einem mittleren Molekulargewicht im Bereich von 2150 bis 3400.

6. Verdickungsmittel-Zusammensetzung, die ein Polyäther nach einem der vorangegangenen Ansprüche enthält.

**Claims**

1. A polyether having the general formula

$$R_1O(CH_2CH_2O)_n(CH_2\underset{\substack{|\\ R_2}}{C}HO)_pH$$

wherein

$R_1$ is a substituted or unsubstituted aliphatic hydrocarbon group having 10–22 carbon atoms or an alkyl phenyl group with an alkyl chain of 7–15 carbon atoms,

$R_2$ is an alkyl group having 8–30 carbon atoms,

n is 40–80 and

p is 1–3.

2. A polyether according to Claim 1, wherein $R_1$ is a tallow group.

3. A polyether according to either Claim 1 or Claim 2, wherein $R_2$ is an alkyl group having 10–18 carbon atoms.

4. A polyether according to any one of the preceding Claims, wherein p has a value of 1.

5. A polyether according to Claim 4, with an average molecular weight of from 2150 to 3400.

6. A thickening agent composition which contains a polyether according to any one of the preceding Claims.

**Revendications**

1. Polyéther de formule générale:

$$R_1O(CH_2CH_2O)_n(CH_2\underset{\substack{|\\ R_2}}{C}HO)_pH$$

dans laquelle:

$R_1$ représente un groupe hydrocarboné aliphatique substitué ou non substitué, renfermant de 10 à 12 atomes de carbone ou un groupe alkylphényle dont la chaîne alkyle renferme de 7 à 15 atomes de carbone,

$R_2$ représente un groupe alkyle renfermant de 8 à 30 atomes de carbone;

n est compris entre 40 et 80; et

p est égal à 1, 2 ou 3.

2. Polyéther selon la revendication 1, dans lequel $R_1$ représente un groupe de suif.

3. Polyéther selon l'une quelconque des revendications 1 ou 2, dans lequel $R_2$ représente un groupe alkyle renfermant de 10 à 18 atomes de carbone.

4. Polyéther selon l'une quelconque des revendications précédentes, dans lequel p est égal à 1.

5. Polyéther selon la revendication 4, dont le poids moléculaire moyen est compris entre 2150 et 3400.

6. Composition d'épaississant contenant un polyéther selon l'une quelconque des revendications précédentes.

Figur 1

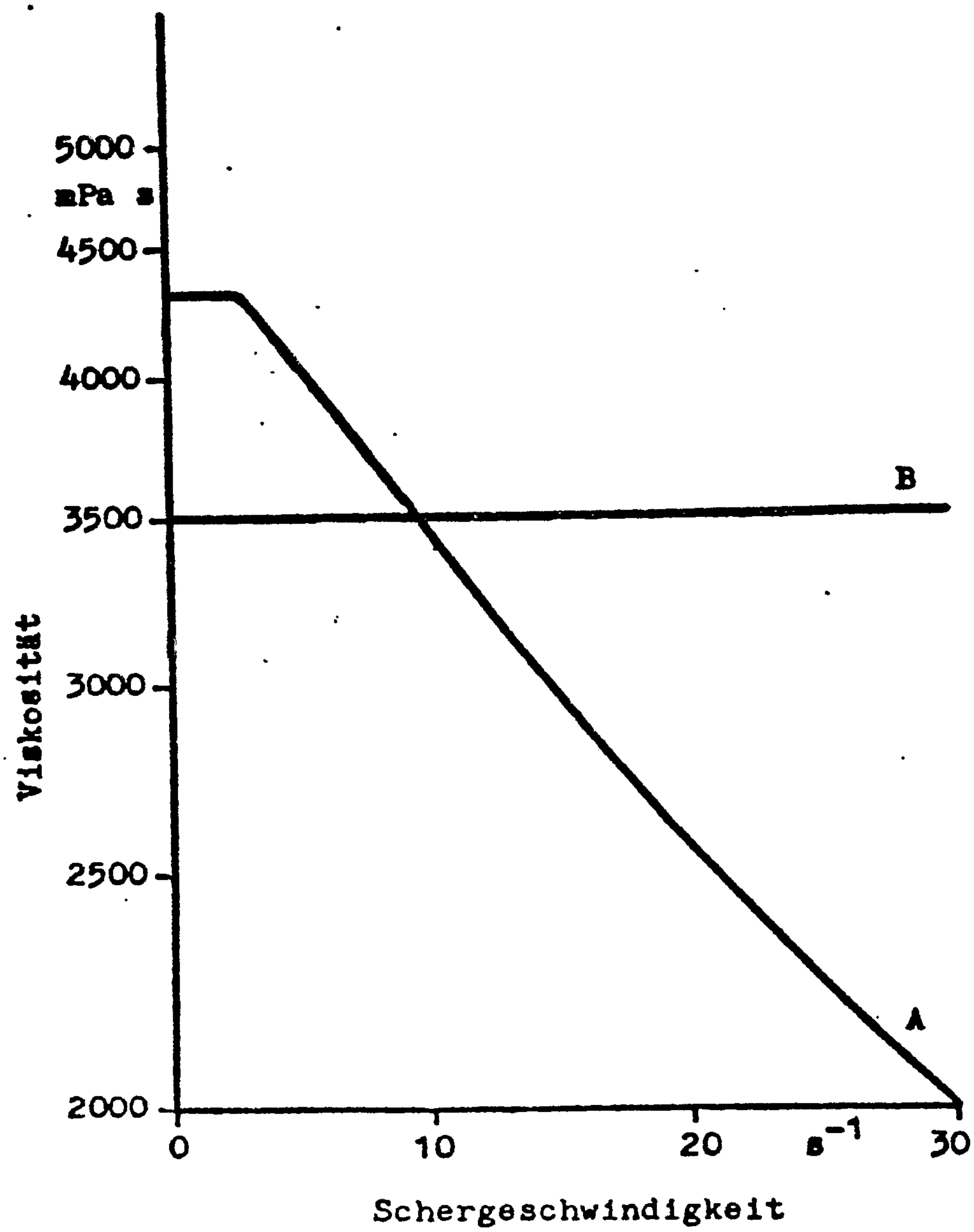
5000
mPa s
4500
4000
3500
3000
2500
2000
Viskosität
B
A
0
10
20
s-1
30
Schergeschwindigkeit